# EUROPÄISCHE PATENTSCHRIFT

(11) **EP 2 129 774 B1**
(45) Veröffentlichungstag und Bekanntmachung des Hinweises auf die Patenterteilung: **13.06.2012**
(21) Anmeldenummer: 08709114.6
(22) Anmeldetag: 20.02.2008
(51) Int. Cl.: A61K 35/34

(54) **ZELLEN ZUR THERAPIE DES HERZENS**
CELLS FOR HEART TREATMENT
CELLULES POUR LE TRAITEMENT CARDIAQUE

(30) Priorität: 20.02.2007 DE 102007008650
(43) Veröffentlichungstag der Anmeldung: 09.12.2009
(73) Patentinhaber: Charité - Universitätsmedizin Berlin, 10117 Berlin (DE)
(72) Erfinder: HAAG, Marion, 13353 Berlin (DE); RINGE, Jochen, 16547 Birkenwerder (DE); SITTINGER, Michael, 12305 Berlin (DE); TSCHÖPE, Carsten, 10715 Berlin (DE)
(74) Vertreter: Schulz Junghans
(86) Internationale Anmeldenummer: PCT/EP2008/052027
(87) Internationale Veröffentlichungsnummer: WO 2008/101936

(56) Entgegenhaltungen:
- WO-A-99/49015
- WO-A-2005/012510
- GOUMANS M-J ET AL: "Human cardiac progenitor cells are able to differentiate into cardiomyocytesin vitro" CIRCULATION, AMERICAN HEART ASSOCIATION, DALLAS, TX, Bd. 112, Nr. 17, SUPPL. S, 16. November 2005 (2005-11-16), Seite U106, XP008090751 ISSN: 0009-7322
- BELTRAMI ANTONIO P ET AL: "Adult cardiac stem cells are multipotent and support myocardial regeneration" CELL, CELL PRESS, CAMBRIDGE, NA, US, Bd. 114, Nr. 6, 19. September 2003 (2003-09-19), Seiten 763-776, XP002304004 ISSN: 0092-8674
- OH H ET AL: "Cardiac progenitor cells from adult myocardium: homing, differentiatin, and fusion after infarction" PROCEEDINGS OF THE NATIONAL ACADEMY OF SCIENCES OF USA, NATIONAL ACADEMY OF SCIENCE, WASHINGTON, DC, Bd. 100, Nr. 21, 14. Oktober 2003 (2003-10-14), Seiten 12313-12318, XP002984517 ISSN: 0027-8424
- VAN VLIET PATRICK ET AL: "Isolation and expansion of resident cardiac progenitor cells" EXPERT REVIEW OF CARDIOVASCULAR THERAPY, FUTURE DRUGS, LONDON, GB, Bd. 5, Nr. 1, 1. Januar 2007 (2007-01-01), Seiten 33-43, XP008090693 ISSN: 1477-9072
- BOYLE ANDREW J ET AL: "Is stem cell therapy ready for patients? Stem Cell Therapy for Cardiac Repair. Ready for the Next Step ." CIRCULATION 25 JUL 2006, Bd. 114, Nr. 4, 25. Juli 2006 (2006-07-25), Seiten 339-352, XP002487309 ISSN: 1524-4539
- HAAG M ET AL: "Isolation and characterization of cardiac-derived progenitor cells from human heart biopsies" TISSUE ENGINEERING, Bd. 13, Nr. 7, Juli 2007 (2007-07), Seite 1720, XP002487310 & CONFERENCE OF THE TISSUE-ENGINEERING-AND-REGENERATIVE-MEDICI NE-INTERN ATIONAL-SOCIETY (TERMIS-EU); LONDON, UK; SEPTEMBER 04 -07, 2007 ISSN: 1076-3279
- BARILE LUCIO ET AL: "Endogenous cardiac stem cells" PROGRESS IN CARDIOVASCULAR DISEASES, Bd. 50, Nr. 1, Juli 2007 (2007-07), Seiten 31-48, XP002487311 ISSN: 0033-0620

## Beschreibung

Die Erfindung betrifft Zellen und eine Zellzubereitung zur Behandlung von Herzerkrankungen, sowie Verfahren zur Herstellung der erfindungsgemäßen Zellen und Zellzubereitungen.

Erkrankungen des Herz- und Kreislaufsystems gehören zu den wichtigsten Krankheitsursachen in industrialisierten Gesellschaften. Darunter ist die Herzinsuffizienz, als Ursache oder Folge einer durch andere Faktoren bedingten Pathologie, eine der am häufigsten auftretenden Erkrankungen. Die Fallzahlen in Europa allein liegen im zweistelligen Millionenbereich.

Zu den experimentellen Therapien, welche im Zusammenhang mit Herzinsuffizienz diskutiert werden, gehört auch die Zelltherapie. Grundlage des therapeutischen Ansatzes ist dabei die Erwartung, dass der geschwächte Herzmuskel durch das Einwandern und die Proliferation von therapeutisch applizierten Zellen in den Herzmuskel gestärkt und seine Funktionstüchtigkeit erhöht würde.

Die Gewinnung von adulten Stammzellen aus dem Herz ist dabei ein Gegenstand der Forschung. Während lange Zeit die Regenerierbarkeit von Herzgewebe grundsätzlich in Frage gestellt wurde, sind zurzeit mehrere Ansätze zur Regeneration von geschädigtem Herzmuskel entweder aus Stammzellen oder nicht ausdifferenzierten, Stammzell-ähnlichen Zellen in der Untersuchung und zum Teil in der klinischen Entwicklung.

Pittenger et al. haben mesenchymale Stammzellen charakterisiert (Science 284, 143-147); diese zeigen unter anderem einen CD90-positiven Phänotyp.

Wang et al. (International Journal of Cardiology 109 (2006) 74 - 81) zeigen die Differenzierung von mesenchymalen Stammzellen der Ratte zu differenzierten Herzzellen in Ko-Kultur mit ausdifferenzierten Herzzellen. Die dabei gewonnenen Zellen sind ebenfalls CD90-positiv. Ähnliche Ergebnisse finden Moscoso et al. (Transplantation Proceedings, 37, 481- 482 (2005)) in Zellen des Schweins.

Goumans et al. (2005, Circulation, American Heart Association, Dallas TX, 112, Nr. 17, Supply. S, U106) zeigt die Isolation von menschlichen foetalen Herz-Vorläuferzellen, die in CD34- und CD45 negative sowie CD105 positive Kardiomyozyten differenzieren.

Beltrami et al. (Cell (2003) 114, 763-776) zeigen Lin(-) c-kit^{POS} Zellen, die Charakteristika von myogenen Vorläuferzellen des adulten Myokardiums aufweisen. Diese sind negativ hinsichtlich der Expression kardialen Myosins und Desmins, Desmins der glatten Muskelzellen, und negative hinsichtlich CD34 und CD45.

Oh et al. (Proc. Nat. Acad. Sci. USA (2003) 100, 12313-12318) zeigen adulte kardiale Progenitorzellen des Herzens, die CD34, CD45 und c-kit negativ sind und kein Myosin exprimieren.

Van Vliet et al. (Future Drugs (2007) 5, 33-43) referieren zu kardialen Vorläuferzellen, darunter sca-1 oder c-kit exprimierende Vorläuferzellen.

WO 2005 012510 A1 zeigt aus Herzbiopsien abgeleitete Zellen, die c-kit und CD34 positiv sind.

WO 99/49015 A2 zeigt Myosin-negative, aus Herzgewebe abgeleitete Stammzellen.

Messina et al. (Circulation Research, Oct. 29, 2004, pp. 911-924; WO2005/012510) beschreiben ein Verfahren zur Isolation und Kultivierung von Herzzellen aus Biopsien. Die dort isolierten Zellen sind unter anderem c-kit/CD117-positiv.

Ein wesentliches Problem bei der Anwendung von Zelltherapie zur Behandlung von Herzerkrankungen ist es, ausreichende Zellmengen zur therapeutischen Anwendung zu erhalten.

Der vorliegenden Erfindung liegt das Problem zugrunde, in einem einfachen Verfahren aus relativ einfach zugänglichem Zellmaterial außerhalb des Körpers eine Zellzubereitung zu gewinnen, welche geeignet ist, einem an Kardiomyopathie und anderen Herzerkrankungen, wie z.B. Infarkten oder deren Folgeerscheinungen, leidenden Patienten mit dem Ziel der Verbesserung seiner Herzleistung zugeführt zu werden.

Erfindungsgemäß wird dieses Problem durch die Zelle, die Zellzubereitung und das Verfahren gemäß den unabhängigen Ansprüchen gelöst.

Gemäß einer Ausführungsform der vorliegenden Erfindung kann eine Zelle bzw. eine Zubereitung einer Vielzahl von Zellen zur Therapie einer Herzerkrankung bereitgestellt werden, indem ein einem Patienten entnommenes Biopsat bzw. eine Gewebeprobe aus Zellen eines Patienten in eine Primärkultur gebracht und über mehrere Passagen vermehrt werden. Bevorzugt ist die Gewinnung der Zellen aus einer Primärkultur, welche aus einer Herzmuskelbiopsie angelegt wurde, beispielsweise eines Stecknadelkopf-großen oder noch kleineren Biopsats. Die Biopsate werden mit Hilfe einer kleinen "Zange" "gerupft". Die Kulturbedingungen sind in Beispiel 1 beschrieben.

Gemäß einer Ausführungsform der vorliegenden Erfindung unterscheidet sich das Verfahren von dem von Messina et al. (siehe oben) beschriebenen Verfahren durch den Verzicht von Cardiotrophin und Thrombin im Zellkulturmedium.

Weiterhin wird kein 2-Mercaptoethanol im Medium verwendet. Gemäß einer Ausführungsform der vorliegenden Erfindung werden die aus dem Biopsat ausgewachsenen Zellen abtrypsiniert. Es entfallen somit alle von Messina et al. beschriebenen Schritte zur "Zellernte". Die Zellen werden nicht in beschichteten Zellkulturplatten ausgesät, da diese Zellen sehr gut adhärieren. Die Weiterkultivierung der Zellen unterscheidet sich dann auch deshalb, weil die bei Messina et al. beschriebenen "Cardiospheres" nicht adhärieren, das heißt, sie wachsen in Kultur nicht auf festen Oberflächen wie z.B. dem Boden einer Zellkulturschale, einer Wand einer Zellkulturflasche, einer Folie in Nährmedium oder einer porösen Matrix für die Zellkultur an. Bevorzugte Zellen der vorliegenden Erfindung adhärieren.

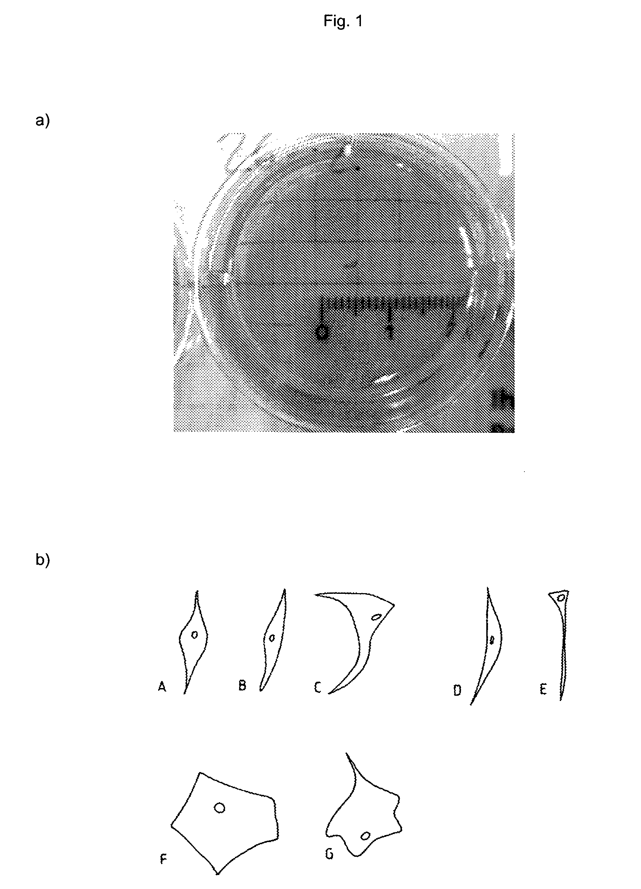

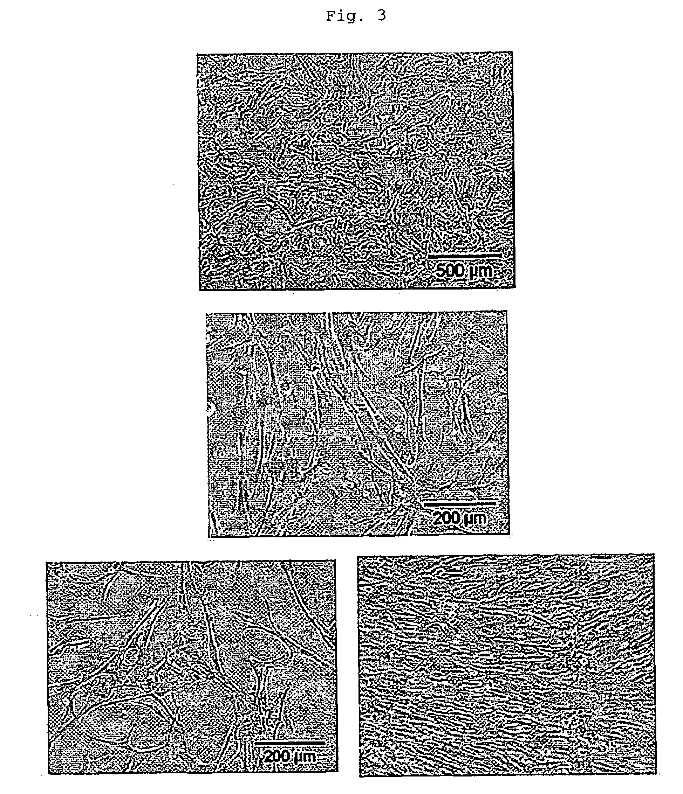

Gemäß einer Ausführungsform der vorliegenden Erfindung wird ein Verfahren bereitgestellt, welches die Gewinnung einer Zellpopulation erlaubt, die sich durch eine Reihe besonderer Eigenschaften auszeichnet: es handelt sich um fibroblastenartige Zellen, d.h. lang gestreckte, spindelförmige Zellen mit einer Morphologie wie in Fig. 1 b) und in Fig. 3 gezeigt, und sie wachsen unter den in Beispiel 1 genannten Zellkulturbedingungen adhärent.

Überraschenderweise sind diese Zellen negativ in Bezug auf die Färbung mit dem für mesenchymale Stammzellen und Fibroblasten charakteristischen Marker CD90.

Es ist dabei offensichtlich, dass bei Anlage einer Primärkultur beispielsweise aus einem Herzbiopsat keine homogene Zellpopulation gewonnen werden kann. Ebenfalls offensichtlich ist, dass für die therapeutische Anwendung zurzeit die Applikation einer Vielzahl von Zellen in Frage kommt, also einer Zellzubereitung. Eine solche Zellzubereitung kann durch ein Zellkulturverfahren, wie in Beispiel 1 beschrieben, gewonnen werden. Die darin enthaltenen Zellen werden sich in ihrem Phänotyp voneinander unterscheiden, da sie, wie oben angeführt, nicht aus einer homogenen Population ausgewachsen sind. Die charakteristischen Merkmale einer erfindungsgemäßen Zellzubereitung sind dem entsprechend nicht absolut als "positiv" oder "negativ" bezüglich eines Zellmarkers anzugeben, sondern in Bezug auf die gesamte Zellpopulation.

So kann sich gemäß einer Ausführungsform der vorliegenden Erfindung die Zellzubereitung dadurch auszeichnen, dass eine Mehrheit der von ihr umfassten Zellen CD90-negativ ist. Dies bedeutet, dass von den in dieser Population umfassten Zellen mindestens 50% durch einen üblichen Farbmarker, z.B. den in den Beispielen angegebenen Farbmarker, im FACS nicht mehr als typischerweise dem Fachmann als CD90-negativ bekannte Zellen gefärbt werden. CD90 (Thy-1) ist ein Marker für Thymuszellen, hämatopoietische Stammzellen, NK-Zellen und Entothelzellen, Fibroblasten und Myofibroblasten. Bevorzugt ist gemäß einer Ausführungsform der vorliegenden Erfindung die Zellzubereitung zu mindestens 80%, noch bevorzugter zu mindestens 90% CD90-negativ. Noch weiter bevorzugt sind Zellzubereitungen, welche zu mehr als 95%, 98% oder 99% CD90-negativ sind.

Um zu einer bevorzugten Homogenität hinsichtlich der CD90-Negativität der Zellzubereitung zu gelangen, kann gemäß einer Ausführungsform der vorliegenden Erfindung das Verfahren der Gewinnung der Zellzubereitung einen Reinigungsschritt umfassen, in welchem die Zellen hinsichtlich ihrer Expression von CD90 selektiert werden. Es sind dazu z.B. Verfahren durch fluoreszenz-basierte Zellsortierung (FACS-Sorting) in geeigneten Geräten bekannt. Dabei werden die mit einem fluoreszenzmarkierten Antikörper gegen das jeweilige Antigen markierten Zellen automatisch in einer Kapillare in eine negative und eine positive Population getrennt.

Weiterhin ist die Trennung durch magnetische Separation bekannt. Dabei werden die Zellen durch Retention der Antigen-positiven Zellen mit Antikörper-gekoppelten Magnetpartikeln in einem sehr starken Magnetfeld getrennt.

Mittel und Verfahren zur Trennung von Zellen hinsichtlich ihrer Expression von Antigenen sind der Fachwelt bekannt. Ebenfalls kann gemäß einer Ausführungsform der vorliegenden Erfindung die Zellzubereitung durch ein Verfahren gewonnen werden, welches alternativ zu oder zusätzlich zu der Trennung nach CD90-Negativität noch weitere Trennschritte umfasst, die nach weiteren möglichen Charakteristika der Zellzubereitung selektieren:

So kann sich gemäß einer Ausführungsform der vorliegenden Erfindung die Zellzubereitung dadurch auszeichnen, dass eine Mehrheit der von ihr umfassten Zellen CD105-positiv ist. Dies bedeutet, dass von den in dieser Population umfassten Zellen mindestens 50% durch einen üblichen Farbmarker, z.B. den in den Beispielen angegebenen Farbmarker, im FACS gefärbt werden. CD105 ist ein für Endothelzellen und mesenchymale Zellen typischer Marker. Bevorzugterweise ist gemäß einer Ausführungsform der vorliegenden Erfindung die Zellzubereitung zu mindestens 90%, noch bevorzugter zu mindestens 98% CD105-positiv.

Weiterhin kann sich gemäß einer Ausführungsform der vorliegenden Erfindung die Zellzubereitung dadurch auszeichnen, dass eine Mehrheit der von ihr umfassten Zellen CD117-negativ ist. Dies bedeutet, dass von den in dieser Population umfassten Zellen mindestens 50% durch einen üblichen Farbmarker, z.B. den in den Beispielen angegebenen Farbmarker, im FACS nicht mehr als typischerweise dem Fachmann als CD117-negativ bekannte Zellen (negative Kontrolle) gefärbt werden. CD117 (c-kit) ist ein Stammzellmarker. Bevorzugterweise ist gemäß einer Ausführungsform der vorliegenden Erfindung die Zellzubereitung zu mindestens 60%, noch bevorzugter zu mindestens 70% CD117-negativ.

Ebenfalls kann sich gemäß einer Ausführungsform der vorliegenden Erfindung die Zellzubereitung dadurch auszeichnen, dass eine Mehrheit der von ihr umfassten Zellen CD166-positiv ist. Dies bedeutet, dass von den in dieser Population umfassten Zellen mindestens 50% durch einen üblichen Farbmarker, z.B. den in den Beispielen angegebenen Farbmarker, im FACS gefärbt werden. CD166 ist das Acronym für "activated leukocyte cell adhesion molecule (ALCAM)", ein für mesenchymale Stammzellen aus dem Knochenmark typischer Marker. Bevorzugter Weise ist gemäß einer Ausführungsform der vorliegenden Erfindung die Zellzubereitung zu mindestens 60%, noch bevorzugter zu mindestens 70% CD166-positiv.

Weiterhin kann sich gemäß einer Ausführungsform der vorliegenden Erfindung die Zellzubereitung dadurch auszeichnen, dass eine Mehrheit der von ihr umfassten Zellen CD34-negativ und CD45-negativ ist. Dies bedeutet, dass von den in dieser Population umfassten Zellen mindestens 50% durch einen üblichen Farbmarker, z.B. den in den Beispielen angegebenen Farbmarker, im FACS nicht mehr als typischerweise dem Fachmann als CD34-negativ bzw. CD45-negativ bekannte Zellen (negative Kontrolle) gefärbt werden. CD34 und CD45 sind Stammzellmarker. Bevorzugterweise ist die Zellzubereitung zu mindestens 60%, noch bevorzugter zu mindestens 70% CD34- und CD45-negativ.

Weiterhin kann sich gemäß einer Ausführungsform der vorliegenden Erfindung die Zellzubereitung dadurch auszeichnen, dass eine Mehrheit der von ihr umfassten Zellen nach einer myogenen Induktion (siehe Beispiele) Desmin-positiv ist, und/oder positiv für Antikörper gegen das Myosin des glatten Herzmuskels ist. Bevorzugt ist die Zellzubereitung zu mindestens 60%, noch bevorzugter zu mindestens 70% Desmin-positiv und/oder Myosin-positiv.

Neben der genannten Charakterisierung als CD90-negativ können gemäß einer Ausführungsform der vorliegenden Erfindung die Zellzubereitungen alle genannten Charakteristika hinsichtlich des Anteils von CD105-positiven, CD117-negativen, CD166-positiven, CD34/45-negativen, Zellen aufweisen.

Hierbei kann sich die Aussage der "Positivität" oder "Negativität" auf zwei verschiedene Populationen beziehen. Dies soll am Beispiel einer als "60% CD105-positiv, 90% CD90-negativ" bezeichneten Zellzubereitung erläutert werden:
- einmal kann diese Bezeichnung sich auf eine Zellzubereitung beziehen, bei welcher 60% *aller* Zellen CD105-positiv sind. Ebenso würde die Charakterisierung 90% CD90-negativ sich auf die Gesamtpopulation beziehen. Da sich die Definition der Positivität oder Negativität hier jeweils auf die ganze Population bezieht, soll dieses Kriterium als "globale Charakterisierung" bezeichnet werden.
- Zum anderen kann eine Zellzubereitung, bei welcher 60% der CD90-negativen Zellen CD105-positiv sind, so bezeichnete werden. Diese Charakterisierung, bei welchem ein zweites Kriterium nur auf Zellen angewendet wird, welche das erste Kriterium erfüllen, soll hier "kumulative Charakterisierung" genannt werden.

Die in Zellzubereitungen enthaltene Zellmenge, welche sicher CD90-negativ und CD105-positiv ist, unterscheidet sich möglicherweise, je nachdem ob die globale oder die kumulative Charakterisierung als Definition verwendet wird. Dieser Unterschied wird um so größer sein, je mehr Kriterien hinzukommen, besonders bei Kriterien nah der 50%.

Gemäß einer Ausführungsform der vorliegenden Erfindung soll eine Zellzubereitung sowohl durch die genannten Kriterien hinsichtlich ihrer Expression von CD90, CD105, CD117, CD166 und CD34/45 sowohl in globaler als auch in kumulativer Charakterisierung definiert sein, und zwar durch alle genannten Prozentzahlen, wobei die kumulative Charakterisierung bevorzugt ist.

Das Verfahren ermöglicht es erstmals, Zellzubereitungen mit einer Zellmenge von über 10¹⁰, sogar bis zu 10¹³⁻¹⁴ Zellen zur therapeutischen Anwendung am Herzen zu erhalten.

Erfindungsgemäß können die Zellen als pharmazeutische Zubereitung dem Patienten verabreicht werden. Zunächst ist hierbei bevorzugt, menschlichen Patienten autologe Zellzubereitungen zu verabreichen. Der Anwendung heterologer Zellzubereitungen stehen die bekannten immunologischen Probleme entgegen; jedoch sind durchaus Indikationen realistisch, in denen auch heterologe Zellzubereitungen z.B. der Transplantation eines Spenderherzens vorzuziehen sind. Insbesondere weisen die Zellzubereitungen den großen Vorteil auf, dass z.B. Zelloberflächenproteine, welche die Erkennung der heterologen Zellen durch das Immunsystem des Patienten ermöglichen, während des Gewinnungsverfahrens gezielt blockiert werden können, so dass die heterologe Zellzubereitung die bekannten immunologischen Nachteile eines heterologen Transplantats nicht oder signifikant vermindert aufweist.

Zur Applikation der erfindungsgemäßen Zellen steht eine Vielzahl bekannter Methoden zur Verfügung. Unter anderem beschreiben Gyöngyösi et al. (J. Kardiol 2004, 11 (Supp B; pp 22-24) die Injektion von Zellen durch ein intramyokardiale katheterbasierte Injektion direkt in den Herzmuskel. Da Fibroblasten Tropismus aufweisen, ist weiterhin eine systemische Applikation vorstellbar.

### Definitionen

Zellen im Sinne dieser Erfindung sind Körperzellen von Säugetieren, insbesondere von Menschen. Erfindungsgemäß können Zellen und Zellzubereitungen gemäß den unabhängigen Ansprüchen zur Zubereitung eines Medikaments zur Behandlung von Krankheiten verwendet werden. Dabei ist ebenso die Zubereitung einer autologen als auch einer allogenen Zelle oder Zellzubereitung möglich. Autologe Zellen oder Zellzubereitungen sind durch Abnahme eines Biopsats aus demselben Patienten gewonnen wurden, dem sie auch zurückgegeben werden. Allogene Zellen oder Zellzubereitungen sind aus einem fremden Patienten gewonnen worden.

Zellen oder Zellzubereitungen können auch aus einem Biopsat gewonnen werden, welches einem Spenderherzen entnommen wurde. Diese Zellen können dann extrakorporal vermehrt und aufbewahrt werden, um im Falle einer medizinischen Notwendigkeit dem transplantierten Patienten gegeben zu werden.

Die Bezeichnung einer Zellpopulation als "fibroblastenartig" im Sinne der vorliegenden Erfindung bedeutet, dass die Zellen in der Mehrzahl im Mikroskop eine im Wesentlichen spindelförmige Form zeigen. Spindelförmig bedeutet, dass die erfindungsgemäßen Zellen in der Mehrzahl eine lang gestreckte Form aufweisen, beispielsweise, dass die Zellen bei Konfluenz eine Länge von 150-250 µm aufweisen. Bei geringerer Konfluenz findet man jedoch auch Zellen mit einer Größe von nur 60 µm bis hin zu über 350 µm (langgestreckte Form). Die Breite der Zellen kann von 13-20 µm reichen, wobei auch 9 µm und über 30 µm möglich sind.

Die Zellen sind auch durch ihre charakteristische Form gekennzeichnet, wie sie in Fig. 1 b) (A-E) und Fig. 3 gezeigt ist. Weiterhin sind fibroblastenartige Zellen im Sinne der vorliegenden Erfindung dadurch gekennzeichnet, dass sie in Kultur gemäß den in den Beispielen angegebenen Zellkulturbedingungen am Boden des Zellkulturgefäßes anhaften oder adhärieren und durch Trypsinierung vom Boden des Zellkulturgefäßes zu lösen sind. Je konfluenter (dichter) die Zellkultur ist, desto einheitlicher fibroblastenartig ist die Zellform.

Die Bezeichnung einer Zelle als "negativ" im Bezug auf einen Gewebsmarker wie z.B. ein Protein der CD ("ctuster of differentiation")-Reihe, z.B. CD90, CD 105, CD117, CD166, bedeutet, dass die Zelle durch vorschriftsmäßige Färbung mit einem markierten Antikörper gegen den bezeichnenden Marker nicht in der Weise gefärbt wird, dass die Zelle im FACS (fluoreszenzbasiertes Zellsortierungsgerät) oder Fluoreszenzmikroskop ein größenordnungsmäßig vergleichbar starkes Signal hinsichtlich der Farbmarkierung des jeweiligen Antikörpers gibt, wie eine von der Fachwelt als "positiv" im Bezug auf den jeweiligen Oberflächenmarker angesehene Zelle mit dem gleichen Antikörper und unter vergleichbaren Bedingungen der Färbung.

Gleiches gilt analog für Färbemethoden innerhalb der Zelle.

Bekannte, unmissverständliche Beispiele für positiv bzw. negativ hinsichtlich zweier wesentlicher Marker im Zusammenhang der vorliegenden Erfindung bezeichnete Zellarten sind mesenchymale Stammzellen. Diese sind positiv für CD 105 und CD 90 und negativ für CD 34 und 45.

Gemäß einer Ausführungsform der vorliegenden Erfindung wird eine primäre, einem lebenden oder vor weniger als 24h verstorbenen Organismus entnommene Gewebeprobeprobe in geeigneter Weise, wie dem Fachmann bekannt, in einer Zellkulturschale unter geeigneten Bedingungen, z.B. bei 37 Grad Celsius, 95% Luftfeuchtigkeit und 5% CO2, in einem Nährmedium in Kultur genommen. Dieser Schritt der "Inkulturnahme" beinhaltet einen partiellen Verdau der Gewebeprobeprobe durch Proteasen. Bevorzugt sind hierbei Trypsin-EDTA und Collagenase IV. Alternativ kann der Verdau auch ohne Trypsin-EDTA vorgenommen werden.

Diese Kultur wird als "Primärkultur" bezeichnet. Die Zellen werden in ihrem Wachstum in regelmäßigen Abständen beobachtet und nach Erreichen einer festgelegten Zelldichte, geerntet/isoliert. Gemäß einer Ausführungsform der vorliegenden Erfindung wachsen die Zellen aus dem Biopsat aus (Auswachskultur) und werden dann geerntet bzw. isoliert. Diese Zellen werden wiederum in Kultur gebracht und immer dann passagiert, wenn die Zellen den Boden des Zellkulturgefäßes zu 70 - 90 % bedecken (Konfluenz von 70-90%).

Das Transferieren von Zellen von einem Kulturgefäß in ein anderes, wobei meist eine Verdünnung der Zellen erfolgt, wird als Passage bezeichnet. Dieser Ausdruck ist synonym mit Subkultur und sollte nicht verwechselt werden mit der Passage in der Virologie (Toni Lindl, "Zell- und Gewebekultur" 4. Auflage, Spektrum Verlag, S. 255). Typischerweise werden dabei die Zellen vom Boden des Zellkulturgefäßes durch Anwendung von Trypsin abgelöst ("Trypsinierung") und mit einer Dichte von 5000 - 6000 Zellen/ cm² wieder ausgesät. Somit gelangen die Zellen von einer Passage in die nächste Passage.

Als "Konfluenz" wird die dichteste mögliche Anordnung von adhärenten Zellen als Monolayer in Kultur bezeichnet (siehe auch Lindl, ibid., S. 253).

Krankheiten, die mit den Zellen, Zellzubereitungen und Medikamenten der unabhängigen Ansprüche behandelt werden können, bzw. Indikationen für die Anwendung der erfinderischen Zellen und Zellzubereitungen können unter anderem Herzleiden wie die ischämische Kardiomyopathie mit guter und schlechter Ejektionsfraktion, inflammatorische Kardiomyopathie mit guter und schlechter Ejektionsfraktion, diastolische Dysfunktion, Aortenklappenfehler (Stenose, Insuffizienz), Mitralklappenfehler (Stenose, Insufflienz), Rechtsherzinsuffizienz, Bradykarde und tachykarde Rhythmusstörungen inkl. AV-Blöcke und Vorhofflimmern, Beschichtung von Koronarstents, Diabetische Kardiopathie, Kollagenosen mit kardialer Beteiligung, Familiäre Kardiomyopathien, viral induzierte Myokarditis und Cor hypertensivum sein.

Gemäß einer Ausführungsform der vorliegenden Erfindung wird eine isolierte Säugetierzelle bereitgestellt, welche durch die folgenden Merkmale gekennzeichnet ist:
- die Zelle ist eine in Zellkultur vermehrte Zelle aus einer Primärkultur einer einem Säugetier entnommenen Gewebeprobe,
- die Zelle ist eine fibroblastenartige Zelle, und
- die Zelle ist CD90-negativ.

Bevorzugt wurde die Zelle durch mindestens drei Passagen in Zellkultur vermehrt. Die Zelle kann humanen Ursprungs sein.

Gemäß einer bevorzugten Ausführungsform ist die isolierte Säugetierzelle CD105-positiv ist. Gemäß einer bevorzugten Ausführungsform ist die isolierte Säugetierzelle CD105-positiv, CD117-negativ, CD166-positiv, CD34/45-negativ, Weiterhin wird ein Verfahren zur Gewinnung einer Zellzubereitung zur Verfügung gestellt, bei welchem
a) eine einem Säugetier entnommene Gewebeprobe einem zeitlich begrenzten Verdau durch ein oder mehrere Bindegewebe verdauende Enzyme unterworfen wird,
b) die zeitlich begrenzt verdaute Gewebeprobe in einem ersten Zellkulturschritt unter zur Zellkultur von Säugetierzellen geeigneten Zellkulturbedingungen in einem Zellkulturmedium in einem Zellkulturgefäß mit einer festen Oberfläche kultiviert wird,
c) aus der Gewebeprobe ausgewachsene, an der festen Oberfläche anhaftende Zellen in einem Passageschritt durch limitierte Proteolyse abgelöst, isoliert sowie in erneut in Zellkulturmedium in verdünnt in Kultur gebracht werden,
d) der Schritt c) mindestens weitere zwei Mal wiederholt wird.

Gemäß einer bevorzugten Ausführungsform umfasst die bei dem Verfahren entnommene Gewebeprobe Herzmuskelgewebe. Noch bevorzugter umfasst die Bindegewebe verdauende Enzymaktivität die Aktivität von Trypsin-EDTA oder Collagenase IV oder eine Kombination beider Aktivitäten und die Dauer des zeitlich begrenzten Verdaus beträgt weniger als 10 Minuten bei einer Aktivität von 0,05 bis 0,25 U/500ml für Trypsin und / oder 0,2 bis 4,5 U/ml für Collagenase IV.

Gemäß einer bevorzugten Ausführungsform des Verfahrens hat der erste Zellkulturschritt eine Dauer von 7 bis 15 Tagen. Bevorzugt ist weiterhin, dass der Passageschritt bei einer Konfluenz der an der festen Oberfläche anhaftenden Zellen von 70% oder größer vorgenommen wird, und/oder dass das Zellkulturmedium des ersten Zellkulturschrittes oder des Passageschrittes oder beider Schritte kein Cardiotropin, kein Thrombin und kein Mercaptoethanol enthält.

Gemäß einer bevorzugten Ausführungsform des Verfahrens wird die in den vorstehend beschriebenen Schritten a bis d gewonnene Zellzubereitung einem Schritt der myogenen Induktion unterworfen, als Folge dessen Zellen gewonnen werden können, die positiv auf Antikörperfärbungen gegen α-Desmin und/oder Myosin reagieren können.

Gemäß einer bevorzugten Ausführungsform des Verfahrens wird die in den Schritten a bis d gewonnene Zellzubereitung einem Reinigungsschritt unterworfen, in welchem
- die die Zellzubereitung umfassenden Zellen mit zur Bindung an spezifische Oberflächenmarker von Zellen befähigten Molekülen in Kontakt gebracht werden und
- diejenigen Zellen, an welche die zur Bindung an spezifische Oberflächenmarker von Zellen befähigten Moleküle gebunden haben, separiert werden.

Dabei können die zur Bindung an spezifische Oberflächenmarker von Zellen befähigten Moleküle Antikörper gegen CD90, CD105, CD117, CD166, CD34 und/oder CD45 sein.

Im Reinigungsschritt können zur Bindung an spezifische Oberflächenmarker von Zellen befähigten Moleküle an Magnetpartikel gebunden sein und während des Reinigungsschrittes in einem Magnetfeld zurückgehalten werden. Ebenso können die Zellen durch Fluoreszenz-aktivierte Zellsortierung (FACS) getrennt werden.

Ebenso wird eine Zellzubereitung zur Verfügung gestellt, die Zellen enthält, die, CD105-positiv, CD117-negativ, CD166-positiv, CD34/45-negativ, α-Desmin-positiv und/oder Myosin-positiv sind.

Ebenso wird eine durch den vorstehend charakterisierten Reinigungsschritt erhaltbare Zellzubereitung zur Verfügung gestellt. Bevorzugterweise sind die darin umfassten Zellen zu mehr als 90% CD90-negativ, zu mehr als 90% CD105-positiv und zu mehr als 50% CD117-negativ. Noch bevorzugter sind die darin umfassten Zellen zu mehr als 95% CD90-negativ, zu mehr als 95% CD105-positiv, zu mehr als 60% CD117-negativ und zu mehr als 50% CD166-positiv. Weiterhin können die so charakterisierten Zellen der Zellzubereitung noch zu mehr als 90% CD34-negativ und zu mehr als 90% CD45-negativ sein.

Ebenso wird eine Zellzubereitung zur Verfügung gestellt, bei der die darin umfassten Zellen zu mehr als 95% CD90-negativ sind, und der CD90-negative Anteil der Zellzubereitung zu mehr als 90% aus CD105-positiven Zellen besteht. Noch bevorzugter sind die darin umfassten Zellen zu mehr als 95% CD90-negativ, und der CD90-negative Anteil der Zellzubereitung ist zu mehr als 90% CD105-positiv und zu mehr als 50% CD117-negativ. Noch bevorzugter sind die darin umfassten Zellen zu mehr als 98% CD90-negativ, und der CD90-negative Anteil der Zellzubereitung ist zu mehr als 95% CD105-positiv, zu mehr als 60% CD117-negativ und zu mehr als 50% CD166-positiv.

Weiterhin bevorzugt ist eine Zellzubereitung bei der die darin umfassten Zellen zu mehr als 95% CD90-negativ sind, der CD90-negative Anteil der Zellpopulation zu mehr als 90% CD105-positiv ist und der gleichzeitig CD90-negative und CD105-positive Anteil der Zellzubereitung zu mehr als 60% CD117-negativ ist.

Vorstehend charakterisierte Zellen oder Zellzubereitungen können zur Herstellung eines Medikaments zur Behandlung von Herzleiden, bevorzugt Kardiomyopathie, verwendet werden.

### Figurenbeschreibung:

- Fig. 1: a) zeigt ein Biopsat in einer Zellkulturschale mit untergelegtem Zentimetermaß b) zeigt in A - E schematisch Zellformen, welche die erfindungsgemäßen fibroblastenartigen Zellen aufweisen können. F und G zeigen Zellformen, die sich von den erfindungsgemäßen fibroblastenartigen Zellen unterscheiden.

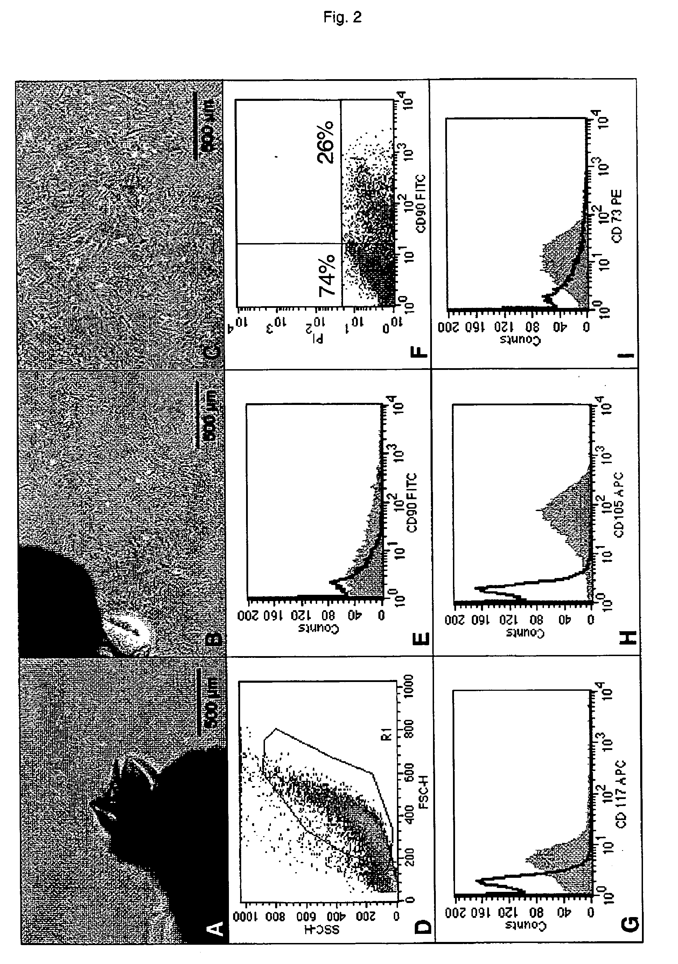

- Fig. 2: zeigt Morphologie und FACS-Analyse der erfindungsgemäßen Zellen bzw. Zellzubereitungen. **A** und **B:** In Kultur auswachsende adhärente Zellen aus (**A:** Kultur nach 5 Tagen), (**B:** Kultur nach 15 Tagen). **C:** Zellkultur der geernteten Zellen in Passage 4 nach 5 Tagen in Kultur. **D-I:** Histogramme von FACS-Analysen, und zwar: **D** Vorwärts- gegen Seitwärtsstreulicht, **E** und **F** FACS-Analyse für CD90, CD117 (**G**), CD105 (**H**) und CD73 (**I**).
- Fig. 3: zeigt photographische Aufnahmen der erfindungsgemäßen fibroblastenartigen Zellen im Mikroskop, und zwar Zellen der Charge 21-1 E4 P1 day6 (oben); 16-1 E2 P3 (Mitte); 17-2 E2 P1 Tag6 (Links unten) und 16-1 E3 P1 konfluent (rechts unten).

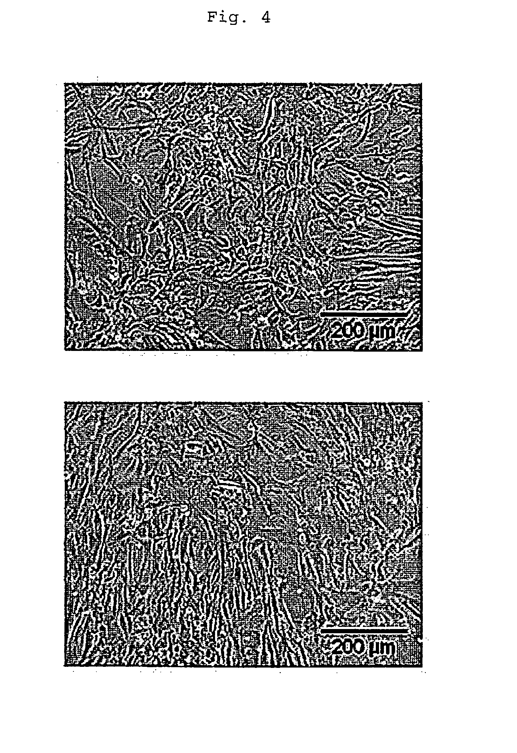

- Fig. 4: zeigt photographische Aufnahmen von Vergleichsbeispielen, welche nicht die Form der erfindungsgemäßen fibroblastenartigen Zellen im Mikroskop aufweisen, und zwar Zellen der Charge 16-4E2 Primar Tag 6 (oben) und 16-1 E3 P3 (Mitte).

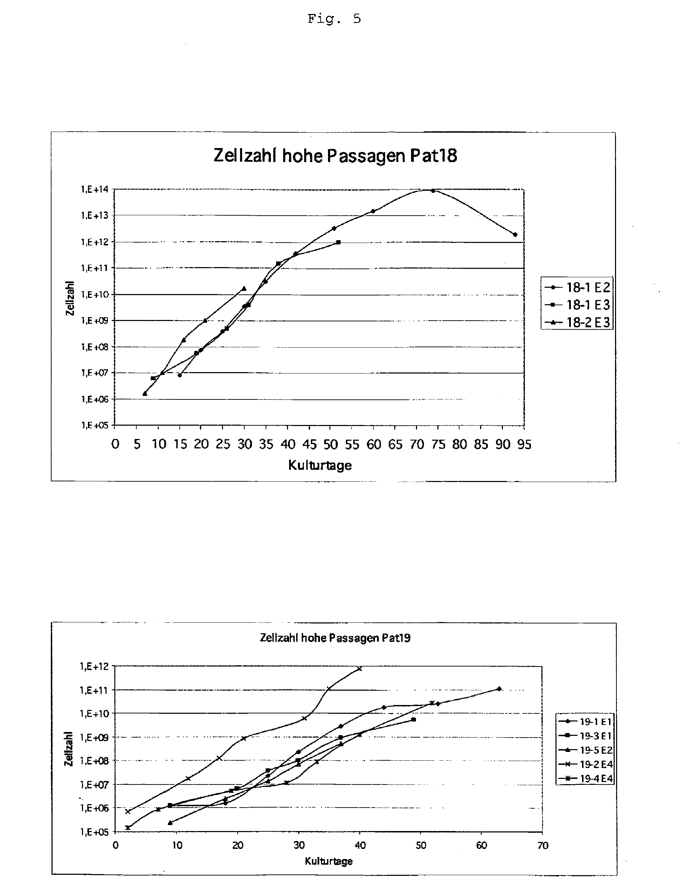

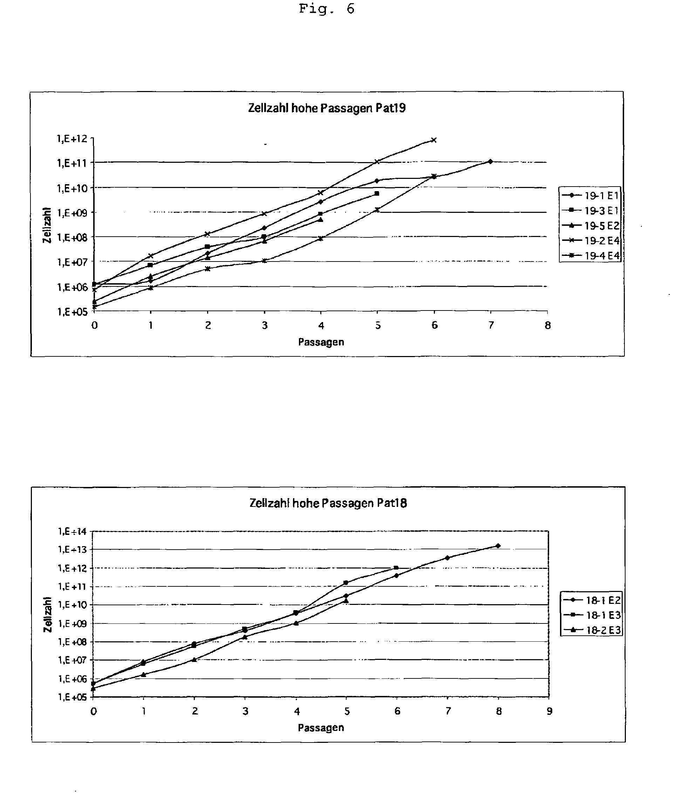

- Fig. 5 und 6: zeigen Beispiele der Wachstumskinetik von Beispielkulturen.

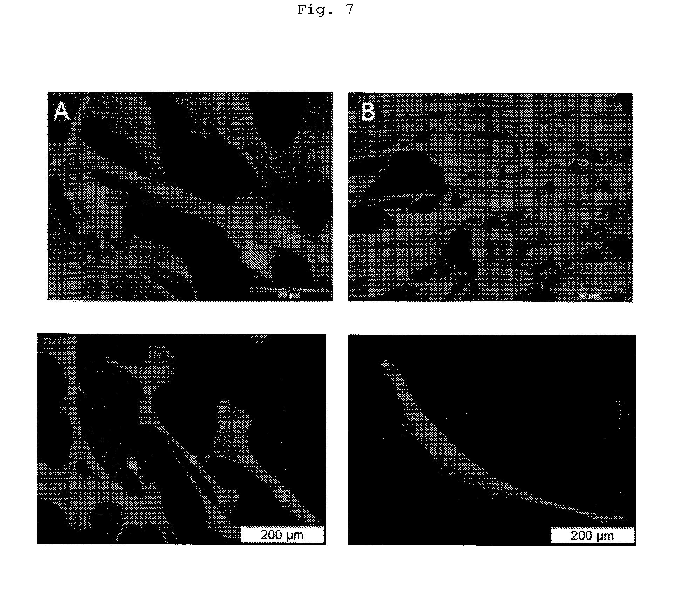

- Fig. 7: zeigt fluoreszensmikroskopische Aufnahmen von Zellen, welche positiv für die Antikörper gegen α-Sarcomeric-Aktin (obere Reihe A - B;, A2172, monoclonaler Anti-Actin (α-Sarcomeric) Antikörper) (Sigma Aldrich) Sekundärantikörper: Ziege α Maus Cy3 (Dianova115-165-003)) sowie gegen den Herzisotyp des Myosins (untere Reihe, gleicher Sekundärantikörper) sind.

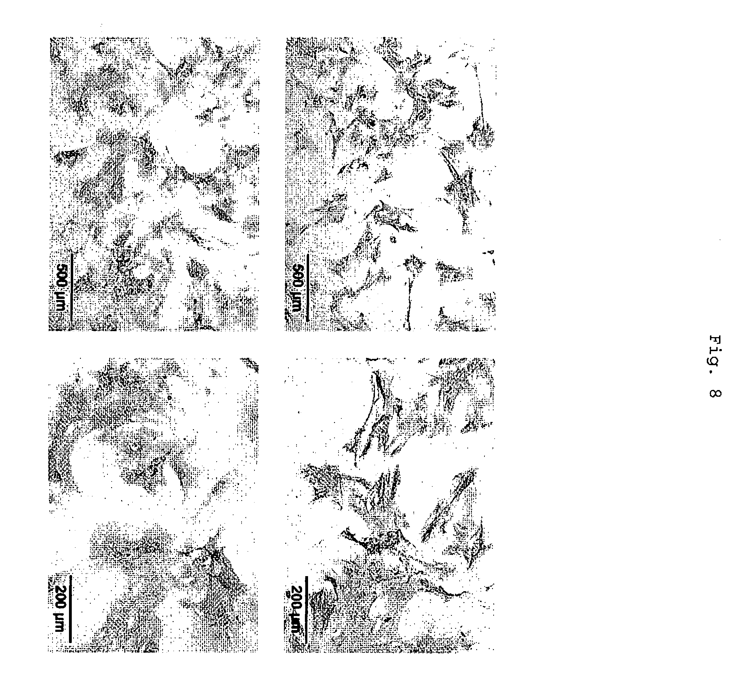

- Fig. 8: zeigt fluoreszensmikroskopische Aufnahmen von Zellen nach einer myogenen Induktion mit 5-Azacytidin, welche positiv für die Antikörper gegen α-Desmin (oben, Monoclonal Maus-anti-menschliches Desmin, Dako) und α-smooth muscle Myosin (unten, Monoclonal Maus-anti menschliches Myosin schwere Kette (smooth muscle), Dako) sind.

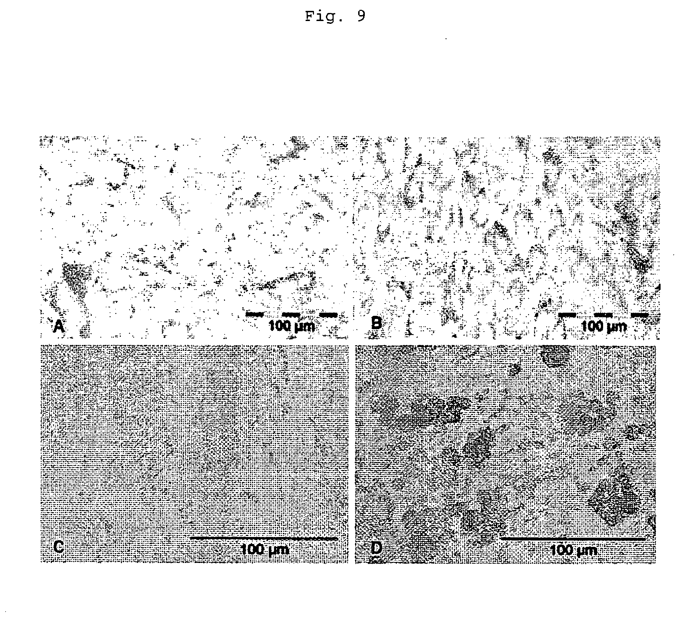

- Fig. 9: zeigt eine Oil Red O Färbung humaner Zellen zum Nachweis der adipogenen Differenzierung oder ihres Ausbleibens. A: cardiale Progenitorzellen Tag 15; B: induzierte cardiale Progenitorzellen Tag 15; C: humane MSC, Tag 15; D: induzierte humane MSC, Tag 15.

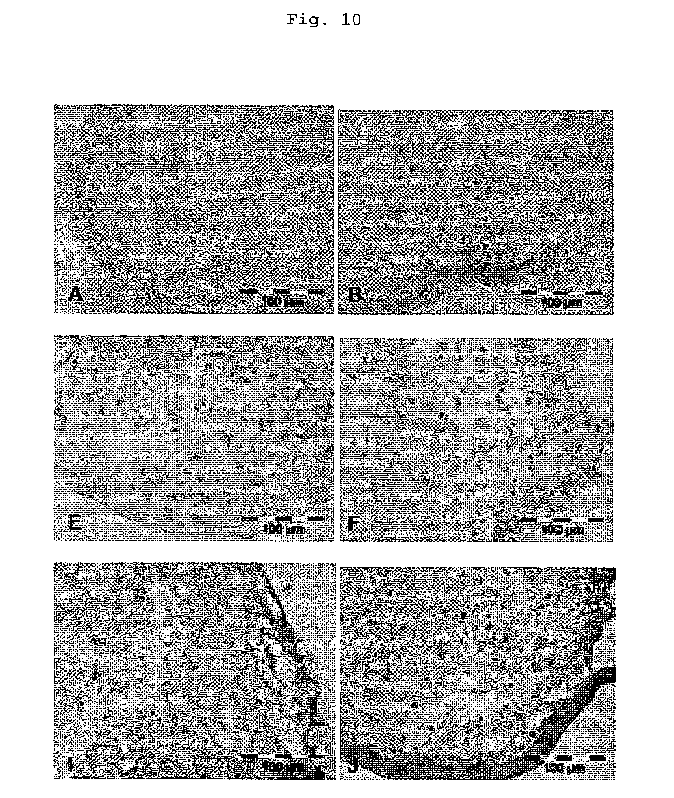

- Fig. 10: zeigt die chondrogene Nachweisfärbung oder deren Ausbleiben beispielhaft an einem Patienten zum Induktionsende (Tag 28); A zeigt die Alcianblaufärbung C die Kollagen Typ II und E die Kollagen Typl Färbung am nicht chondorgen induzierten Zellpellet (Kontrolle). B zeigt die Alcianblau , D die Kollagen Typ II und F die Kollagen Typ I Färbung am chondrogen induzierten Zellpellet.

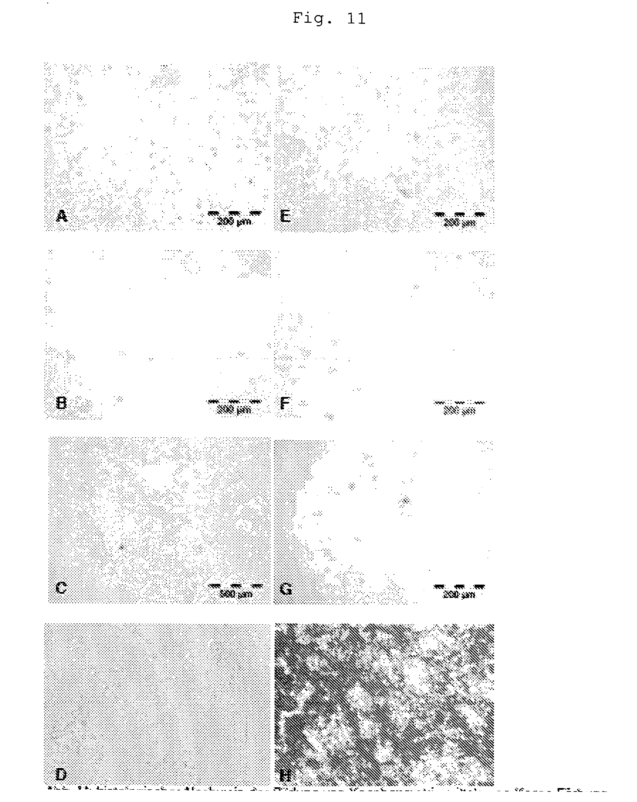

- Fig. 11: zeigt den histologischen Nachweis der Bildung oder des Ausbleibens von Knochenmatrix mittels Kossa-Färbung. A-C zeigen nicht induzierte Kontrollkulturen der Patienten 1-3 an Tag 28, D zeigt eine nicht induzierte humane MSC Kontrollkultur an Tag 28, E-G zeigen osteogen induzierte Kulturen der Patienten1-3 an Tag 28, H zeigt eine deutlich erkennbare Knochenmatrix in einer osteogen induzierten hMSC-Kultur am Tag 28.

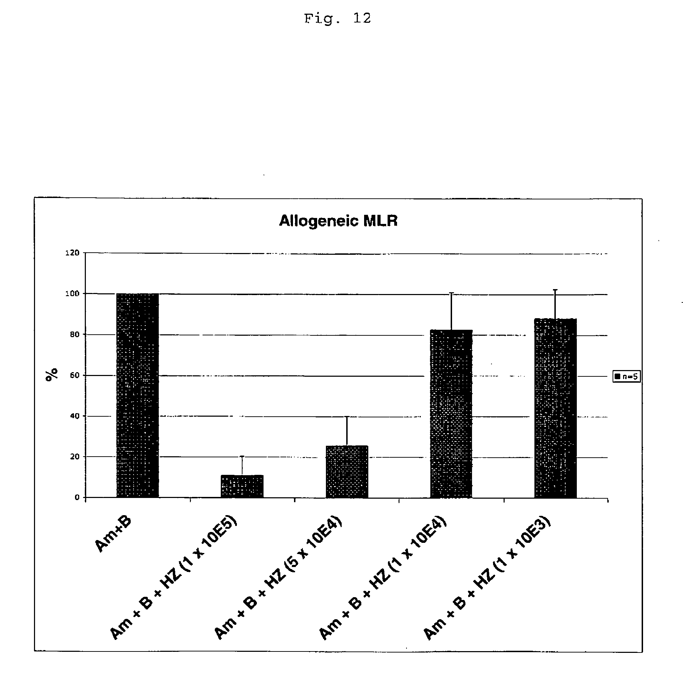

- Fig. 12: Fig 12 zeigt die Auswertung eines Experiments zur gemischten Lymphozytenreaktion bei 5 Patienten (n=5).
- Fig: 13: zeigt die Ergebnisse der FACS-Sortierung: oben vlnr: unsortiert, CD90-positive Zellen; CD90-negative Zellen.

### Beispiele

### Beispiel 1: Kulturbedingungen und Passage

### Aufarbeitung der Biopsate:

Aus einem aus Herzmuskel gewonnenen Biopsat wurden bis zu 5 mm³, bevorzugt 1-2 mm³ große Stücke mit einem sterilen Skalpell geschnitten und mit PBS (Phosphatgepufferte isotone Kochsalzlösung) (kalzium- und magnesiumfrei) gewaschen.
Die so gewonnenen Gewebeproben wurden 3 x 5 min bei 37 °C mit Trypsin/EDTA (1:500 verdünnt, Aktivität der unverdünnten Lösung 0,125-0,15 u/ml; Biochrom AG, Berlin) und 0,45 U/ml Collagenase IV (Sigma Aldrich) in PBS verdaut. Nach jeweils 5 min wurden die Biopsate in neues Trypsin-Collagenasegemisch überführt. Der Überstand wurde verworfen und das angedaute Gewebe mit IMDM (Iscove's Modified Dulbecco's Medium komplettiert mit 10 % FBS, 100 U/ml Penicillin, 100 µg/ml Streptomycin, 2 mmol/L L- Glutamin) gewaschen, anschließend wurden die Explantate in einem Zellkulturgefäß mit 9,6 cm² Wachstumsoberfläche in komplettiertem IMDM kultiviert. Die Explantate müssen am Boden des Kulturgefäßes fixiert werden ("festdrücken").

Je nach Explantat (patientenabhängig) wachsen nach 7-15 Tagen fibroblastenartige, adhärente Zellen aus.

### Ernte der ausgewachsenen Zellen:

Die Explantate/ Zellen wurden mit PBS vorsichtig gewaschen (Explantate dürfen sich nicht ablösen), und anschließend mit 1 ml Trypsin/EDTA (0,05%/0,02%) pro Explanat 3-5 min inkubiert (Kulturplattengröße 9,6cm² , siehe oben) und anschließend die Verdaureaktion mit IDH-Medium (IMDM, DMEM, Ham F-12 Mix komplettiert mit 2% B27, 10 ng/ml epidermal growth factor, 20 ng/ml basic fibroblast growth factor, 3,3 % FKS, 100 U/ml Penicillin, 100 ug/ml Streptomycin, 2 mmol/L L- Glutamin) gestoppt. Anschließend wird das Medium mit den abgelösten Zellen 5 min bei 353 g zentrifugiert, der Überstand wird verworfen und die Zellen in IDH-Medium resuspendiert und in einem Gesamtvolumen von 3 ml Medium (IDH) in einem 9,6cm² Kulturgefäß in Kultur genommen (Bedingungen: 5 % CO2, 37°C 95% Luftfeuchtigkeit).

Alternativ kann ein Medium mit 5% Humanserum statt 3,3 %FKS und ohne Beigabe von B27 verwendet werden. Alternativ kann auch das kommerziell erhältliche Medium Opti pro^{™} (Gibco, 12309) komplettiert mit der entsprechenden Menge Serum, Penicillin/Streptomycin, 200 mM L-Alanyl-Lglutamine (Biochrom, K0302, 20 ml/L), EGF und FGF verwendet werden.

Bei einer Konfluenz von 70-90% werden die Zellen passagiert. Hierzu wird das Medium entfernt, der Zellrasen einmal mit PBS abgespült und die Zellen abtrypsiniert. Es werden dann 5.000-6.000 Zellen pro cm² Zellkulturgefäßoberfläche ausgesät. Die erfindungsgemäßen Zellen bzw. Zellzubereitungen wurden bis zu 4 Mal vom selben Explantat im Abstand von 6-10 Tagen (vom Biopsat abhängig) isoliert.

### Beispiel 2: Färbung

Die trypsinierten Zellen wurden mit PBS/0,5% BSA gewaschen. Anschließend werden 250.000 Zellen in 0,1ml PBS/0,5%BSA und dem entsprechenden Antikörper (AK) 15 min auf Eis inkubiert. Es wurden Fluoresceinisothiocyanat (FITC) markierte, R-Phycoerythrin (PE) markierte und Allophycocyanin (APC) markierte Maus anti-Mensch AK verwendet (siehe Tab. X). Die Zellen wurden nach der Färbung mit PBS/0,5%BSA gewaschen. Apoptotische Zellen wurden mit Propidiumiodid (PI, Sigma, Taufkirchen, Deutschland) markiert, um sie aus der Auswertung auszuschließen. Die Analyse erfolgte am FACSCalibur (Becton Dickinson, Heidelberg, Deutschland) und die Auswertung mit Hilfe der CellQuest Software (Becton Dickinson).

**Tabelle 1: Informationen zu den benutzten Antikörpern**

| Antikörper | Verdünnung | Hersteller | Bestell Nr. |
|---|---|---|---|
| FITC α human CD90 | 1:75 | Pharmingen | 555595 |
| FITC α human CD105 | 1:20 | Acris | SM1177F |
| APC α HumanCD117 | 1:20 | Invitrogen | CD11705 |
| PE α humanCD166 | 1:20 | Pharmingen | 559263 |
| FITC α humanCD45 | 1:100 | Pharmingen | 555482 |
| PE α humanCD34 | 1:50 | Pharmingen | 555822 |
| PE α humanCD73 | 1:20 | Pharmingen | 550257 |

Fig. 2 zeigt in den Panelen **A** und **B** in Kultur wachsende adhärente Zellen aus den Biopsaten aus (**A:** Kultur nach 5 Tagen), die sich strecken und nach einigen Tagen eine fibroblastenartige Form annehmen (**B**: Kultur nach 15 Tagen). **C** zeigt eine Zellkultur der geernteten Zellen in Passage 4 nach 5 Tagen in Kultur. **D-I** zeigen in der FACS-Analyse, dass die Zellen zum größten Teil negativ sind für CD90 (**E** und **F**) und CD117 (**G**) und positiv für CD105 (**H**) und größtenteils auch positiv für CD73 (**I**). Die Linie zeigt das Histogramm der negativen (ungefärbten) Zellpopulation, flächig dargestellt ist das Histogramm der gefärbten Zellen.

Die folgende Tabelle 2 zeigt beispielhaft die bei einer Messung verwendeten Parameter am Gerät "FACSCalibur" der Firma Becton-Dickinson:

**Tabelle 2: Messparameter der Kanäle des FACS-Gerätes**

| Param | Detektor | Spannung (Voltage) | Verstärkung (AmpGain) | Modus |
|---|---|---|---|---|
| P1 | Vorwärtsstreuung | E-1 | 3.27 | Lin |
| P2 | Seitwärtsstreuung | 424 | 1.00 | Lin |
| P3 | Fluoreszenzkanal1 | 505 | 1.00 | Log |
| P4 | FI. 2 | 489 | 1.00 | Log |
| P5 | FI. 3 | 590 | 1.00 | Log |
| P6 | FI. 2-A | | 1.00 | Lin |
| P7 | FI. 4 | 740 | | Log |

**Tabelle 3: Kompensationen der Kanäle des FACS-Gerätes**

| Kompensation |
|---|
| FL1 - 2,5 % FL2 |
| FL2 - 1,0 % FL1 |
| FL2 - 2,0% FL3 |
| FL3 - 14,6% FL2 |
| FL3 - 0,8% FL4 |
| FL4 - 1,0% FL3 |

| |
|---|
| Grenzwert (treshhold) primärer Parameter: Fluoreszenzkanal 1, Wert: 35 |

### Beispiel 3: Kultur mit 5-Azacytidin (Myogene Induktion, Myogenese)

Nach einer Stimulation mit 5-Azacytidin (24 h - 20 µl/ml, 10µM) und einer 4wöchigen Kultivierung sind die Zellen positiv für α-Desmin-Antikörper und α-smooth muscle myosin-Antikörper (siehe Fig. 8).
(nach: Xu W, Zhang Z, Mesenchymal stem cells from adult human bone marrow differentiate into a cardiomyocyte phenotype in vitro, Exp Biol Med (Maywood). 2004 Jul;229(7):623-31)

### Differenzierung in Fett/Knochen/Knorpel ("Multilineage")

Werden die Zellen mit den nach Pittenger et al. (Pittenger et al., Multilineage potential of adult human mesenchymal stem cells.Science. 1999 Apr 2;284(5411):143-7) für die hier beschriebenen Zellen modifizierten Protokollen induziert, differenzieren sie nicht in Richtung Fett, Knochen und Knorpel. Die Protokolle von Pittenger et al. wurden dazu an die in Beispiel 1 verwendeten Medien adaptiert.

### Beispiel 4:. Isolierung einer Zellpopulation per FACS Sortierung

Die Zellen werden analog für Beispiel 2 gefärbt, wobei: 10 - 15 Mio Zellen mit αhumanCD90 FITC (BD) in einer 1:100 Verdünnung markiert werden. Die Zellen werden mit 10 ml PBS/BSA gewaschen und in 1 ml PBS/BSA Arbeitsvolumen aufgenommen.

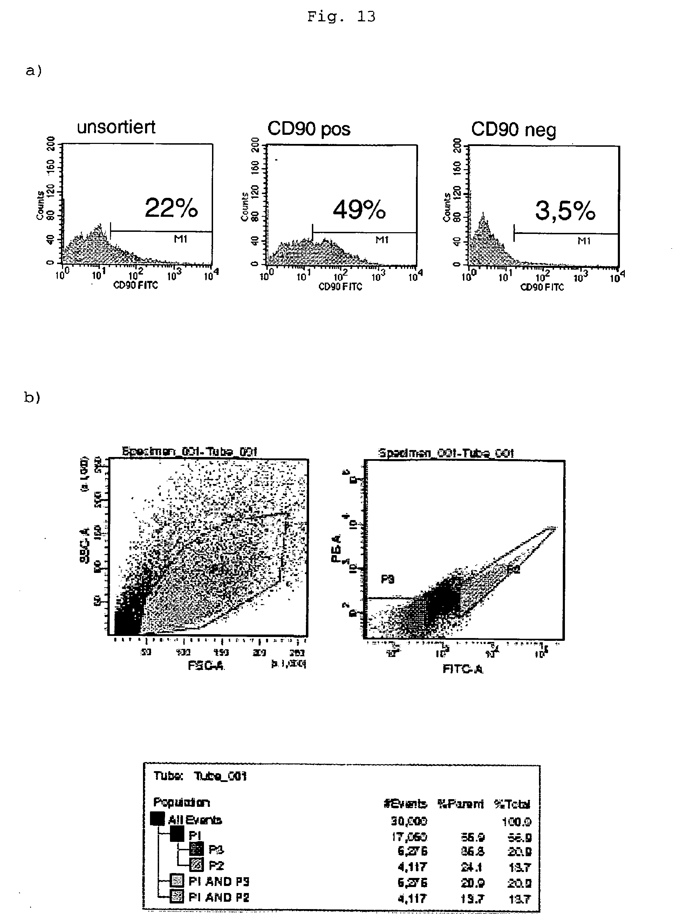

Die so gefärbten Zellen werden nach den bekannten Methoden sortiert, z.B. durch FACS-Sortierung. Das Ergebnis der Sortierung ist in Fig. 13 gezeigt. Die Prozentzahl gibt die Zahl der CD90 positiven Zellen an. Vor der Sortierung sind 22 % der Zellen CD90 positiv, in der auf CD90 sortierten Population waren dann 49% der Zellen CD90 positiv. Die auf CD90 negativ sortierte Population war zu 96,5% negativ.

### Beispiel 5:. gemischte Lymphozytenreaktion (MLR, Mixed Lymphocyte Reaction)

Durch die MLR lassen sich alloreaktive T-Zellen im Experiment nachweisen. Diese sind ein Indiz dafür, dass es zu einer Abstoßung fremden Gewebes/fremder Zellen kommt. Bei der MLR werden Lymphocyten eines Individuums (Spender A) mit Lymphocyten eines anderen Individuums (Spender B) gemischt. Wenn die T-Zellen des einen Individuums die MHC-Moleküle des anderen Individuums als fremd erkennen, proliferieren dessen T-Zellen. Die Proliferation der Zellen des Spenders A wird durch eine Behandlung der Zellen mit Mytomycin C (m), einem Cytostatikum, verhinert.
Die CAP-Zellen besitzen immunmodulatorische Eigenschaften. Wie die MLR zeigt, verhindern sie die Proliferation der T-Zellen bereits ab einem Einsatz von 5 x 10⁴ Zellen, wirken also immunsuppressiv. Somit verhalten sich diese Zellen ähnlich wie mesenchymale Stammzellen im Hinblick auf die MLR. Dies ist ein wichtiges Indiz für die Möglichkeit einer allogene Anwendung der Zellen.
Verschiedene Konzentrationen von Herzzellen (HZ) wurden mit mit Mitomycin behandelten Zellen des Spenders A (Am) gemischt. Bei einer Konzentration von 5 x 10⁴ und 1 x 10⁵ Herzzellen wurde die Proliferation und somit eine Immunreaktion der Zellen des Spenders B unterdrückt. Als Referenz dient die Immunreaktion der Zellen des Spenders A (Mitomycin behandelt, Am) mit den Zellen des Spenders B (siehe Fig. 12).

**Tabelle 4: Antikörper für die FACS Analyse vor MLR**

| AB Bezeichnung | Firma | Produkt-Nummer |
|---|---|---|
| CD31 - PE | BD | 555446 |
| CD34 - PE | BD | 550761 |
| CD45 - FITC | BD | 555482 |
| CD73 - PE | BD | 550257 |
| CD80 - PE | BD | 557227 |
| CD86 - PE | BD | 555653 |
| CD90 - FITC | BD | 555595 |
| CD105 - PE | Caltag | MHCD10504-4 |
| HLA I-FITC | BD | 555552 |
| HLA II-FITC | BD | 555558 |
| CD 40 PE | Serotec | MCA1590PE |

## Patentansprüche

1. Isolierte Säugetierzelle, **gekennzeichnet durch** die folgenden Merkmale:
- die Zelle ist eine in Zellkultur vermehrte Zelle aus einer Primärkultur einer einem Säugetier aus Herzgewebe entnommenen Gewebeprobe,
- die Zelle ist eine fibroblastenartige Zelle,
- die Zelle ist CD90-negativ,
- die Zelle ist CD105-positiv,
- die Zelle ist CD117-negativ und
- die Zelle ist Desmin-positiv und/oder Myosin-positiv.

2. Isolierte Säugetierzelle gemäß Anspruch 1, **dadurch gekennzeichnet, dass** die Zelle durch mindestens drei Passagen in Zellkultur vermehrt wurde.

3. Isolierte Säugetierzelle gemäß einem der vorstehenden Ansprüche, **dadurch gekennzeichnet, dass** die Zelle CD166-positiv, CD34-negativ und / oder CD45-negativ ist.

4. Zellzubereitung umfassend Zellen gemäß einem der Ansprüche 1 bis 3.

5. Zellzubereitung gemäß 4, **dadurch gekennzeichnet dass** die Zellzubereitung folgende Merkmale aufweist: die darin umfassten Zellen sind zu mehr als 90 % CD90-negativ, zu mehr als 90% CD105-positiv und zu mehr als 50% CD117-negativ.

6. Zellzubereitung gemäß einem der Ansprüche 4 bis 5, **dadurch gekennzeichnet, dass** die Zellzubereitung folgende Merkmale aufweist: die darin umfassten Zellen sind zu mehr als 95% CD90-negativ, zu mehr als 95% CD105-positiv, zu mehr als 60% CD117-negativ und zu mehr als 50% CD166-positiv.

7. Zellzubereitung gemäß einem der Ansprüche 4 bis 6, **dadurch gekennzeichnet, dass** die Zellzubereitung folgende Merkmale aufweist: die darin umfassten Zellen sind zu mehr als 95% CD90-negativ, und der CD90-negative Anteil der Zellzubereitung ist zu mehr als 90% CD105-positiv.

8. Zellzubereitung gemäß einem der Ansprüche 4 bis 7, **dadurch gekennzeichnet, dass** die Zellzubereitung folgende Merkmale aufweist: die darin umfassten Zellen sind zu mehr als 95% CD90-negativ, und der CD90-negative Anteil der Zellzubereitung ist zu mehr als 90% CD105-positiv und zu mehr als 50% CD117-negativ.

9. Zellzubereitung gemäß einem der Ansprüche 4 bis 7, **dadurch gekennzeichnet, dass** die Zellzubereitung folgende Merkmale aufweist: die darin umfassten Zellen sind zu mehr als 98% CD90-negativ, und der CD90-negative Anteil der Zellzubereitung ist zu mehr als 95% CD105-positiv, zu mehr als 60% CD117-negativ und zu mehr als 50% CD166-positiv.

10. Zellzubereitung gemäß einem der Ansprüche 4 bis 9, **dadurch gekennzeichnet, dass** die Zellzubereitung folgende Merkmale aufweist: die darin umfassten Zellen sind zu mehr als 95% CD90-negativ, der CD90-negative Anteil der Zellpopulation ist zu mehr als 90% CD105-positiv und der gleichzeitig CD90-negative und CD105-positive Anteil der Zellzubereitung ist zu mehr als 60% CD117-negativ.

11. Verwendung von Zellen gemäß Anspruch 1 bis 3, oder einer Zellzubereitung gemäß Anspruch 4 bis 10, zur Herstellung eines Medikaments zur Behandlung von Herzleiden.

## Claims

1. Isolated mammalian cell, **characterized by** the following features:
- the cell is a cell propagated in cell culture from a primary culture of a tissue sample obtained from heart tissue of a mammal,
- the cell is a fibroblast-like cell,
- the cell is CD90 negative,
- the cell is CD105 positive,
- the cell is CD117 negative and
- the cell is desmin positive and/or myosin positive.

2. Isolated mammalian cell according to claim 1, **characterized in that** the cell was propagated in cell culture for at least three passages.

3. Isolated mammalian cell according to one of the preceding claims, **characterized in that** the cell is CD166 positive, CD34 negative and / or CD45 negative.

4. Cell preparation comprising cells according to one of claims 1 to 3.

5. Cell preparation according to claim 4, **characterized in that** the cell preparation comprises the following features: the cells comprised therein are more than 90 % CD90 negative, more than 90% CD105 positive and more than 50% CD117 negative.

6. Cell preparation according to one of claims 4 to 5, **characterized in that** the cell preparation comprises the following features: the cells comprised therein are more than 95% CD90 negative, more than 95% CD105 positive, more than 60% CD117 negative and more than 50% CD166 positive.

7. Cell preparation according to one of claims 4 to 6, **characterized in that** the cell preparation comprises the following features: the cells comprised therein are more than 95% CD90 negative, and the CD90 negative portion of the cell preparation is more than 90% CD105 positive.

8. Cell preparation according to one of claims 4 to 7, **characterized in that** the cell preparation comprises the following features: the cells comprised therein are more than 95% CD90 negative, and the CD90 negative portion of the cell preparation is more than 90% CD105 positive and more than 50% CD117 negative.

9. Cell preparation according to one of claims 4 to 7, **characterized in that** the cell preparation comprises the following features: the cells comprised therein are more than 98% CD90 negative, and the CD90 negative portion of the cell preparation is more than 95% CD105 positive, more than 60% CD117 negative and more than 50% CD166 positive.

10. Cell preparation according to one of claims 4 to 9, **characterized in that** the cell preparation comprises the following features: the cells comprised therein are more than 95% CD90 negative, the CD90 negative portion of the cell population is more than 90% CD105 positive and the portion of the cell preparation that is both CD90 negative and CD105 positive is more than 60% CD117 negative.

11. Use of cells according to claims 1 to 3, or of a cell preparation according to claims 4 to 10, for the manufacture of a medicament for the treatment of heart disease.

## Revendications

1. Cellule de mammifère isolée, **caractérisée par** les caractéristiques suivantes:
- la cellule est une cellule multipliée en culture cellulaire à partir d'une culture
- primaire d'un échantillon tissulaire prélevé sur le tissu cardiaque d'un mammifère,
- la cellule est une cellule de type fibroblaste,
- la cellule est CD90 négative,
- la cellule est CD105 positive,
- la cellule est CD117 négative et
- la cellule est desmine positive et/ou myosine positive.

2. Cellule de mammifère isolée selon la revendication 1, **caractérisée en ce que** la cellule a été multipliée par au moins trois passages en culture cellulaire.

3. Cellule de mammifère isolée selon une des revendications précédentes, **caractérisée en ce que** la cellule est CD166 positive, CD34 négative et/ou CD45 négative.

4. Préparation cellulaire comprenant des cellules selon une des revendications 1 à 3.

5. Préparation cellulaire selon la revendication 4, **caractérisée en ce que** la préparation cellulaire présente les caractéristiques suivantes : les cellules qui y sont comprises sont à plus de 90% CD90 négatives, à plus de 90 % CD105 positives et à plus de 50 % CD117 négatives.

6. Préparation cellulaire selon une des revendications 4 à 5, **caractérisée en ce que** la préparation cellulaire présente les caractéristiques suivantes : les cellules qui y sont comprises sont à plus de 95% CD90 négatives, à plus de 95 % CD105 positives, à plus de 60 % CD117 négatives et à plus de 50 % CD166 positives.

7. Préparation cellulaire selon une des revendications 4 à 6, **caractérisée en ce que** la préparation cellulaire présente les caractéristiques suivantes : les cellules qui y sont comprises sont à plus de 95% CD90 négatives, et la fraction CD90 négative de la préparation cellulaire est à plus de 90 % CD105 positive.

8. Préparation cellulaire selon une des revendications 4 à 7, **caractérisée en ce que** la préparation cellulaire présente les caractéristiques suivantes : les cellules qui y sont comprises sont à plus de 95% CD90 négatives, et la fraction CD90 négative de la préparation cellulaire est à plus de 90 % CD105 positive et à plus de 50 % CD117 négative.

9. Préparation cellulaire selon une des revendications 4 à 7, **caractérisée en ce que** la préparation cellulaire présente les caractéristiques suivantes : les cellules qui y sont comprises sont à plus de 98% CD90 négatives, et la fraction CD90 négative de la préparation cellulaire est à plus de 95 % CD105 positive, à plus de 60 % CD117 négative et à plus de 50 % CD166 positive.

10. Préparation cellulaire selon une des revendications 4 à 9, **caractérisée en ce que** la préparation cellulaire présente les caractéristiques suivantes : les cellules qui y sont comprises sont à plus de 95% CD90 négatives, la fraction CD90 négative de la population cellulaire est à plus de 90 % CD105 positive et la fraction simultanément CD90 négative et CD105 positive de la préparation cellulaire est à plus de 60 % CD117 négative.

11. Utilisation de cellules selon les revendications 1 à 3, ou d'une préparation cellulaire selon les revendications 4 à 10, pour la fabrication d'un médicament destiné au traitement d'affections cardiaques.
